# EUROPÄISCHE PATENTSCHRIFT

(11) **EP 0 490 281 B1**
(45) Veröffentlichungstag und Bekanntmachung des Hinweises auf die Patenterteilung: **04.09.1996**
(21) Anmeldenummer: 91120935.1
(22) Anmeldetag: 06.12.1991
(51) Int. Cl.: C07H 21/00, C12Q 1/68

(54) **3'- Amino- oder thiolmodifizierte, Fluoreszenzfarbstoff-gekoppelte Nukleoside, Nukleotide und Oligonukleotide sowie ein Verfahren zur Herstellung und ihre Verwendung**
3'- Amino or thiolmodified, fluorescence coupled nucleoside and oligonucleotide, a method for their preparation and their use
3'- Amino ou thiolmodifié, fluorescence couplé nucléoside et oligonucléotide, leur procédé de preparation et leur utilisation

(30) Priorität: 11.12.1990 DE 4039488
(43) Veröffentlichungstag der Anmeldung: 17.06.1992
(73) Patentinhaber: HOECHST AKTIENGESELLSCHAFT, 65926 Frankfurt am Main (DE)
(72) Erfinder: Engels, Joachim, Prof. Dr., W-6242 Kronberg (DE); Herrlein, Mathias, W-6000 Frankfurt am Main 71 (DE); Konrad, Renate, W-6231 Sulzbach am Taunus (DE); Mag, Matthias, W-6370 Oberursel (DE)

(56) Entgegenhaltungen:
- WO-A-88/00201
- GB-A- 2 236 852

## Beschreibung

Markierte Oligonukleotide haben in der Gentechnologie außerordentlich viele Anwendungen gefunden, da ihre Handhabung leichter ist als die der herkömmlich als Hybridisierungsproben verwendeten DNA-Sonden, die durch Restriktionsverdau aus nativem Genmaterial hergestellt werden.

Markierte Oligonukleotide, die in Form von sogenannten "antisense"-DNA-Oligonukleotiden eingesetzt werden, können regulierend in das Zellgeschehen eingreifen und gewinnen damit z. B. für die in vivo Untersuchung der Expression von Proteinen immer größere Bedeutung. Der Mechanismus läuft nach heutiger Kenntnis über DNA-DNA-, DNA-RNA- und RNA-RNA-Wechselwirkungen ab, ist aber im einzelnen noch nicht geklärt.

In vitro dienen markierte Oligonukleotide z. B. der Identifizierung von Genfragmenten innerhalb einer Genbank, indem man mit Hilfe des markierten Oligonukleotids geblottete Genproben der Genbank sondiert und identifiziert.

Um solche Versuche in vitro oder in vivo durchführen zu können, müssen die Oligonukleotide, wie schon erwähnt, markiert werden. Neben der radioaktiven Markierung durch geeignete Isotope werden als Form einer nichtradioaktiven Markierung schon derivatisierte Fluoreszenzfarbstoffe verwendet, die die Möglichkeit einer leichteren und ungefährlicheren Handhabung bieten. Aus der WO 88/00201 sind beispielsweise in 2'- oder in 5'-Position fluoreszenzfarbstoffmarkierte Oligonukleotide bekannt, welche aus dem entsprechenden, in 2'- bzw. 5'-Position aminosubstituierten Oligonukleotiden hergestellt werden.

Bislang wurde eine solche Technik auch schon erfolgreich zur nichtradioaktiven Sequenzierung von DNA angewendet. Hier sind im wesentlichen auf der Methode von Sanger (F. Sanger, S. Nicklen und S. Coulson, Proc. Natl. Acad. Sci. USA 74, 5463 (1977)) basierende Ansätze durchgeführt worden.

Das Fluoreszenzlabel wird entweder am 5'-Ende des Oligonukleotids (L. E. Hood, L. M. Smith und C. Heiner, Nature 321, 674 (1986)) oder an der Nukleobase (J. M. Prober, G. L. Trainor und R. J. Dam, Science 238, 336 (1987)) angebracht (G. L. Trainor, Anal. Chem. 62, 418 (1990)). Ein entscheidender Nachteil des zuletzt angeführten Verfahrens beruht darauf, daß das Fluoreszenzlabel während der Synthese, d. h. während der Polymerisation und hier speziell während der enzymatischen Polymerisation eingeführt wird. Dieser Verfahrensschritt hat zur Konsequenz, daß nur noch einige Polymerasen für die Synthese eingesetzt werden können, daß die Akzeptanz der Triphosphate durch die Polymerasen sinkt und daß außerdem ein starker Substrat-Oberschuß notwendig ist.

Die Einführung eines Fluoreszenzlabels ist jedoch nicht auf die Sanger-Sequenzierung beschränkt. Auch die chemische Sequenzierung nach Maxam-Gilbert mit Fluoreszenzlabel ist bekannt (H. Voss, C. Schwager, U. Wirkner, B. Sproat, J. Zimmermann, A. Rosenthal, H. Erfle, J. Stegemann und W. Ansorge, Nucl. Acids Res. 17, 2517 (1989)).

Analog dazu ist auch die Kartierung von Restriktionsfragmenten mit Fluoreszenzdetektoren beschrieben (A.V. Carrano, J. Lamerdin, L.K. Ashworth, B. Watkins, E. Branscomb, T. Slezak, M. Raff, P.J. de Jong, D. Keith, L. McBride, S. Meister, M. Kronick, Genomics 4, 129 (1989) und S. Brenner und K.J. Livak, Proc. Nati. Acad. Sci. USA 86, 8902 (1989)).

Es wurde nun gefunden, daß ein Fluoreszenzfarbstoff über eine Amino- oder Thiolgruppe in 3'-Position (in α- oder β-Stellung) eines Nukleosids, Nukleotids oder Oligonukleotids gekoppelt und diese Verbindung vorteilhaft für Synthese von Gegensträngen in Anwesenheit eines Template-Stranges oder von Oligonukleotiden sowie für die Detektion von genetischem Material in vivo und in vitro angewendet werden kann.

Die Erfindung betrifft somit:
1. Einen Stoff der Formel I 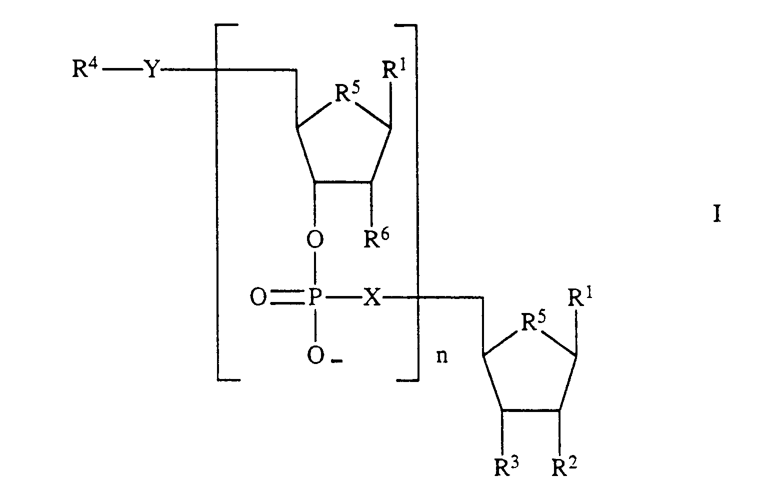 in der
   - R¹: eine Purin- oder Pyrimidinbase ist,
   - R³: ein über eine Amino- oder Thiolgruppe gebundener Fluoreszenzfarbstoff in α- oder β-Stellung und
   - R²: Wasserstoff, eine Hydroxyl-, geschützte Hydroxyl- oder Methoxygruppe in α- oder β-Stellung bedeuten,
   - n: eine Zahl von ≧ 0 ist,
   - R⁴: eine 5'-Schutzgruppe oder Phosphat, Pyrophosphat, Triphosphat ist,
   - R⁵: Sauerstoff, Fluormethylen, Difluormethylen oder Methylen ist,
   - R⁶: eine Hydroxyl- oder Methoxygruppe oder Wasserstoff in α- oder β-Stellung, bedeutet, wobei R¹, R⁵ und R⁶ jeweils gleiche oder unterschiedliche Bedeutungen haben können,
   Y und X Sauerstoff, Schwefel, NH oder Methylen sind
   wobei X und Y jeweils gleich oder verschieden sein können.
2. Ein Verfahren zur Herstellung der unter 1, charakterisierten Verbindung, dadurch gekennzeichnet, daß die in 3'-Stellung befindliche OH-Gruppe des Nukleosids, Nukleotids oder Oligonukleotids zu einer Amino- oder Thiolgruppe derivatisiert und anschließend ein Fluoreszenzfarbstoff angekoppelt wird.
3. Die Verwendung der unter 1. charakterisierten Verbindungen bei
   a) der Synthese von Gegensträngen in Anwesenheit eines Template-Stranges,
   b) der Synthese von definierten Oligonukleotiden,
   c) ihrer Detektion in vivo und in vitro und
   d) der Delektion von Nukleinsäuren in vivo und in vitro.

Im folgenden wird die Erfindung detailliert beschrieben, insbesondere in ihren bevorzugten Ausführungsformen, Ferner wird die Erfindung durch den Inhalt der Ansprüche bestimmt.

Die Verbindungen der allgemeinen Formel I werden im wesentlichen nach literaturbekannten Methoden synthetisiert (M. Gait, Oligonucleotide Synthesis, IRL-Press, Oxford 1984).

Für die Kopplung des Farbstoffs an der 3'-Position geht man von einer Verbindung der Formel II, vorzugsweise von einem Nucleosid aus.

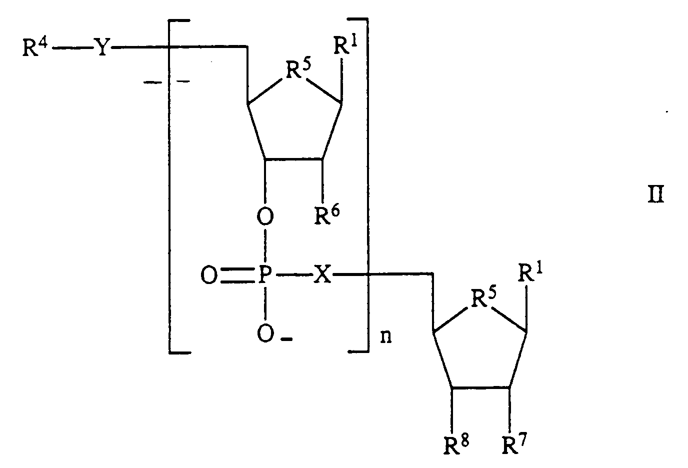

Die Verbindung der Formel II, in der R¹-R⁶, X, Y, und n die oben genannten Bedeutungen haben, wobei die Substituenten gleich oder verschieden sein können und R⁷ oder R⁸ Wasserstoff, eine Hydroxyl- oder geschützte Hydroxyl- oder Methoxygruppe bedeuten, wird für die Kopplung eingesetzt. Die Farbstoffkopplung erfolgt über die 3'-ständige Hydroxylgruppe durch Einführung eines Amins oder Thiols.

Zur Einführung des Azids wird in 3'-Position eine Leaving-Gruppe eingeführt. Als Leaving-Gruppe gilt vorzugsweise Triflat oder Mesylat oder Tosylat. Durch einen nukleophilen Angriff mit Azid, vorzugsweise Lithiumazid erfolgt die Einführung des Azids. Der nukleophile Angriff eines Thiolats oder S-geschützten Thiolats ergibt das Thiol oder das geschützte Thiol.
Die gewünschte Stereochemie am Zuckerteil des Nukleosids läßt sich am besten durch Sₙ2-Substitutionen erzielen.

Die Aminogruppe läßt sich einfach über das Azid und die anschließende Reduktion zum Amin erhalten (Lit.: W.S. Mungall und R.L. Letsinger, J. Org. Chem., Vol. 40, No. 11, 1659 (1975)). Bevorzugt ist in diesem Schritt die Staudingerreaktion mit Triphenylphosphin und Wasser (Lit.: M. May und J.W. Engels, Nucl. Acids Res. 17, 15, 5973 (1989)).

Alle Verbindungen wurden durch NMR, Elementaranalyse, UV, IR etc. in ihrer Konstitution und Konfiguration bestimmt.

Nach Beendigung der Polykondensation kann die freie 3'-Amino- oder Thiolgruppe des Zuckerteils am 3'-Ende der DNA mit einem reaktiven Fluoreszenzfarbstoff nach literaturbekannten Verfahren reagieren (Hunkapiller, Nucl. Acids, Res. 13, 2399, 1 985; Hodges R.R. et al. Biochemistry, Vol. 28, 261 (1989)).

Alternativ erhält man die Aminogruppe auch über die Mitsunobu-Reaktion (Synthesis, Vol 1, 1 98 1, S. 1) und entsprechend der von Yamamoto et al. (J. Chem. Soc. Perk. Trans. I, 1, 306, 1980) beschriebenen Reaktion.

Die Kupplung des Fluoreszenzfarbstoffs über die Thiolgruppe erfolgt in analoger Art und Weise. Es wird jedoch statt der Azid- eine Thiolgruppe in geschätzter oder ungeschützter Form eingeführt.

Die Kopplung des Fluoreszenzfarbstoffs an die freie Aminogruppe oder Thiolfunktion des Nukleosids, Nukleotids oder Oligonukleotids kann alternativ auch erst nach deren Verwendung erfolgen. Es ist beispielsweise möglich, die Fluoreszenzfarbstoff-Reaktion nach dem Terminationsschritt der DNA- oder RNA-Sequenzierungsreaktion durchzufahren, indem man den Gesamtansatz mit dem Fluoreszenzfarbstoff markiert.

Als Fluoreszenzfarbstoffe können prinzipiell alle handelsüblich erhältlichen Farbstoffe verwendet werden, die mit einer Amino- oder Thiolgruppe reagieren, vorzugsweise Fluoresceine, Rhodamine, Texasrot, NBD (4-Fluoro-7-nitrobenzofurazan von Sigma), Coumarine, Fluorescamine, Succinylfluoreszine und Dansyle.

Das derivatisierte Reaktionsgemisch kann durch eine Gelelektrophorese aufgetrennt und durch Photometrie oder Laserspektroskopie detektiert werden (H. Swerdlow und R. Gesterland, Nucl. Acids Res. 18, S. 1415 (1990)). Als gut einsetzbar hat sich auch die Kapillarelektrophorese (A.S. Cohen et al., P.N.A.S. US. 85, 9660 (1988)), z. B. mit Acrylamidgelen gefüllt, erwiesen. Die Detektion erfolgt dann vorzugsweise am Austritt der Kapillare (H. Swerdlow, S. Wu, H. Harke, N. Dovichi, Chromatography 516, 61 (1990)).

Ausgehend von einer Verbindung der Formel I, die in 5'-Position ein Triphosphat besitzt, kann man mittels eines beliebigen Primers und eines Templatestranges mit Hilfe einer Polymerase, d. h. eines Enzyms, welches in Gegenwart geeigneter Substrate eine getreue, komplementäre Kopie der Sequenz synthetisiert, in Gegenwart der vier Nukleosidtriphosphate die Synthese des DNA-Doppelstranges durchfuhren, vorzugsweise mit Hilfe der T7- oder Taq-Polymerase, der DNA-Polymerase I und der Reversen Transkriptase. Als 5'-Schutzgruppen gelten Trityl, Methoxy- oder Dimethoxytrityl (Gait, Oligonucleotide Synthesis, IRL-Press, Oxford 1984).

Die Termination der Synthese kann jeweils durch Einsatz eines 3'-aminomodifizierten A,C,G,T-Nukleotids der Formel I spezifisch bestimmt werden. Dies ist für die Synthese von DNA-Gegensträngen in Anwesenheit eines Template-Stranges und damit auch für die Sequenzierung von DNA-Strängen von besonderer Bedeutung, da der Einsatz eines modifizierten Nukleotids einen sehr basenspezifischen Abbruch der Reaktion gewährleistet.

Die Synthese von RNA-Nukleosiden, Nukleotiden und Oligonukleotiden erfolgt in analoger Art und Weise.

Darüber hinaus ist die Synthese von derivatisierbaren Oligonukleotiden mit Hilfe eines Startnukleotids, welches am 3'-Ende amino- oder thiovariiert und an einem polymeren Träger fixiert wurde, möglich.

Als Oligonukleotide gelten alle im herkömmlichen Sinn hergestellten DNA- und RNA-Nukleotide, vorzugsweise jedoch in einer Länge von 2 bis 100, besonders bevorzugt 12-50 Nukleotiden (chemische Synthese) bzw. in einer Länge bis ca. 3.000 Nukleotiden (enzymatische Synthese), je nach Effizienz der verwendeten Polymerase.

Die Oligonukleotidsynthese erfolgt, ausgehend von dem Startnukleotid-Träger-Komplex, im herkömmlichen Sinn, d. h. in 3'- und 5'-Richtung und ermöglicht die Synthese eines Oligonukleotids definierter Sequenz.

Die Fixierung des Startnukleosids an handelsüblichen Trägern erfolgt über einen Verbindungsarm (Spacer), der sich nach der Synthese spalten läßt; beispielsweise über die literaturbekannte Bernsteinsäureverknüpfung oder aber über eine Verknüpfung mit Urethan (Efimov et al., Nucl. Acids. Res. 11, 8369, 1983).

Nach erfolgter Synthese muß das Oligonukleotid mit geeigneten Reagenzien vom Träger abgespalten werden. Die Derivatisierung mit einem beliebigen Fluoreszenzfarbstoff erfolgt direkt danach.

Die so synthetisierten und am 3'-Ende modifizierten Oligonukleotide können dann zur Detektion von z. B. komplementären Oligonukleotiden verwendet werden.

Die erfindungsgemäße Verwendung verbindet zwei Vorteile miteinander:
1) Bei der Herstellung eines markierten Gegenstranges erfolgt gleichzeitig der Strangabbruch durch das endständige 3'-amino- oder thiolmodifizierte Nukleotid.
   Durch die Fluoreszenzfarbstoff-Markierung desselben an der 3-Position ist außerdem eine gefahrlose Detektion möglich.
2) Bei der Herstellung von genau definierten Oligonukleotiden ist durch Markierung des an einen Träger gekoppelten und ebenfalls 3'-amino- oder thiolmodifizierten Startnukleotids eine exakte Oligonukleotidsynthese verbunden mil der Möglichkeit zur Fluoreszenzmarkierung und damit zur Detektion möglich.

Die im folgenden aufgeführten Beispiele dienen der weiteren Erläuterung der Erfindung.

### Beispiele

### 1. Anomere 3'-Amino- oder Azido-nucleosid-5'-triphosphate

### Beispiel 1

### Synthese von 5'-Triphosphat-3'-amino-3'-desoxyribosid-Thymidin;

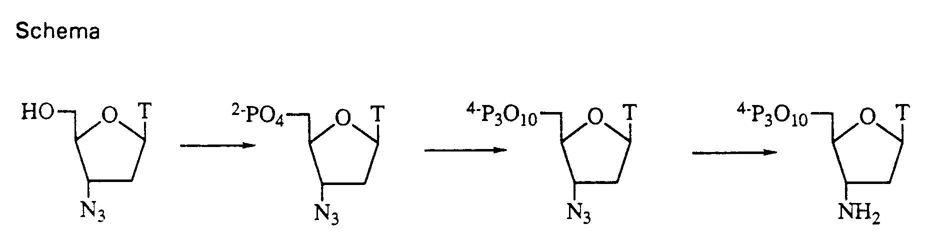

### 5'-Monophosphat-3'-azido-3'-desoxyribosid-thymidin

3'-Azidothymidin (Sigma) (160 mg, 0,6 mmol) wird unter Rühren in 10 ml Triethylphosphat gelöst. Mit einem Tropftrichter setzt man der Lösung bei 40°C 0,2 ml POCl₃ in 6 ml Triethylphosphat zu. Nach 24 h neutralisiert man mit 50 ml gesättigter NaHCO₃-Lösung und schüttelt je zweimal mit 60 ml Toluol und 100 ml Ether aus. Die wäßrige Phase wird mit Aqua dest. auf 500 ml verdünnt und auf eine Anionenaustauschersäule (®Sephadex A-25; 50 x 2,5 cm) gegeben, die durch Überleiten mit 200 ml 0,2 M TBK-Puffer (Triethylammoniumbicarbonat-Puffer; pH 7,5 wurde selbst eingestellt) Mit HCO₃⁻ als Gegenion beladen ist. Dann reinigt man durch Überleiten von 300 ml Aqua dest. und eluiert zuerst mit 200 ml 0,1 M TBK-Puffer und dann mit einem linearen Gradienten von 0,1 - 0,2 M TBK-Puffer (Gradientengesamtvolumen 1350 ml). Im Chromatogramm zeigt sich ein Peak, der Produktpeak. Das Produkt eluierte bei einer Pufferkonzentration von 0,12 bis 0,15 M TBK-Puffer. Die DC-positiven 20 ml-Fraktionen werden vereinigt und durch Gefriertrocknung eingeengt. Das Triethylammoniumbicarbonat entfernt man durch mehrmaligen Zusatz von Ethanol. Das Produkt liegt als weißes, kristallines Triethylammoniumsalz vor.

Ausbeute: 220 mg (56,2 %); MG: 651,81; R_{f} (Ammoniak : Isoprop. : Wasser 10 : 70 :20): 0,40; 300 MHz-¹H-NMR (D₂O): 1,36 (t, 3H, CH₃); 1,90 (s, 3H, CH₃); 2,50 (m, 4H, 2',2''-H); 3,18-3,30 (dd, 2H, CH₂); 4,10 (m, 1H, 4'-H); 4,3 (m, 1H, 3'- H); 6,31 (t, 1 H, 1'-H); 7,7 (s, 1H, 6-H); 11,50 (s, 1H, NH), 300 MHZ-³¹P-NMR (85%ige H₃P0₄ extern, D₂0): 1,23 (s, 1P).

### 5'-Triphosphat-3'-azido-3'-desoxyribosid-Thymidin

5'-Monophosphat-3'-azido-3',2'-desoxyribosid-Thymin als Triethylammoniumsalz (130 mg, 0,2 mmol) wird in 6 ml abs. DMF (Dimethylformamid) gelöst und der Lösung bei 25°C N,N'-Carbonyl-diimidazol (162,15 mg, 1 mmol) in 3 ml abs. DMF unter Rühren zugesetzt. Nach 2 h Reaktionszeit setzt man 1 ml abs. Methanol zu, rührt weitere 15 min und destilliert das Methanol ab. Der Rückstand wird mit 5 ml einer 0,2 M Lösung von Tri-n-butylammonium-Pyrophosphat in DMF versetzt und über Nacht bei Raumtemperatur gerührt. Man filtriert den aus Imidazol-Pyrophosphat bestehenden Niederschlag ab, wäscht mit 20 ml DMF und engt das Filtrat am Rotationsverdampfer ein. Den Rückstand löst man in 250 ml 0,1 M TBK-Puffer und gibt die Lösung auf eine mit HCO₃⁻ beladene Sephadex A-25 Anionenaustauschersäule. Man reinigt das Produkt durch Überleiten von 200 ml Aqua dest. und eluiert mit einem linearen Gradienten von 0 bis 0,5 M TBK-Puffer (Gradientengesamtvolumen 2.000 ml). Im Chromatogramm erscheinen zwei Peaks, von denen der erste durch das Monophosphat und der zweite durch das Produkt erzeugt wird. Das Produkt eluierte bei einer Pufferkonzentration von 0,30 bis 0,35 M TBK-Puffer. Die positiven 20 ml Fraktionen werden vereinigt und durch Gefriertrocknung eingeengt. Das Triethylammoniumbicarbonat entfernt man durch mehrmaligen Zusatz von Ethanol. Das Produkt liegt als weißes, kristallines Triethylammoniumsalz vor.

Ausbeute: 92 mg (50,4 %ig); MG: 911,95; R_{f} (Ammoniak : Isoprop. : Wasser 10 : 70 : 20): 0,14; 300 MHz-³¹P-NMR (85 %ige H₃P0₄ extern, D₂0): -10,74 (d, ²J_{PP} = 22,1 Hz, 1P, Alpha-P-Atom); -11,45 (d, ²J_{PP} = 21,9 Hz, 1P, Gamma-P-Atom); -22,99 (t, ³J_{PP} = 20,3 Hz, 1P, Beta-P-Atom).

### 5'-Triphosphat-3'-amino-3'-desoxyribosid-Thymidin

5'-Triphosphat-3'-azido-3',2'-desoxyribosid-Thymin (68 mg, 0,075 mmol) wird in 5 ml Dioxan-Aqua dest. (2 : 1) gelöst und unter Rühren bei Raumtemperatur mit Triphenylphosphin (200 mg, 0,75 mmol) versetzt. Die Reaktionsmischung wird bei 25°C für 30 h gerührt und danach das Lösungsmittel abgezogen. Den Rückstand löst man in 30 ml Aqua dest. und extrahiert dreimal mit je 30 ml Ether. Die wäßrige Phase verdünnt man auf 150 ml und gibt die Lösung auf eine mit HCO₃⁻ beladene Sephadex A-25 Anionenaustauschersäule. Man reinigt das Produkt durch Überleiten von 200 ml Aqua dest. und eluiert mit einem linearen Gradienten von 0 bis 0,5 M TBK-Puffer (Gradientenvolumen 2.000 ml). Das Chromatogramm zeigt drei Peaks, von denen der erste die Monophosphatverbindung 6, der zweite den Produktpeak und der dritte den Eduktpeak 7 darstellt. Das Produkt eluierte bei einer Pufferkonzentration von 0,40 bis 0,42 M TBK-Puffer. Die positiven 25 ml Fraktionen werden vereinigt und durch Gefriertrocknung eingeengt. Das Triethylammoniumbicarbonat entfernt man durch Zusatz von Ethanol. Das Produkt liegt als weiß-amorphes Triethylammoniumsalz vor.

Ausbeute: 57 mg (85,7 %ig); MG: 885,90; R_{f} (Ammoniak : Isoprop. : Wasser 10 : 70 : 20): 0,08; 300 MHz-¹H-NMR (D₂0): 1,30 (t, 3H, CH₃); 1,92 (s, 3H, CH₃); 2,64 (m, 4H, 2',2''-H); 3,19-3,21 (dd, 2H, CH₂); 4,26 (m, 1H, 4'-H); 4,41 (m, 1H, 3'-H); 6,34 (t, ³J_{HH} = 6,74 Hz, 1H, 1'-H); 7,69 (s, 1H, 6-H); 11,50 (s, 1H, NH). 300 MHz-³¹P-NMR (85 %ige H₃P0₄ extern, D₂0); -10,75 (d, ²J_{PP} = 22 Hz, 1P; Alpha-P-Atom); - 11,45 (d, ²J_{PP} = 21,9 Hz, 1P, Gamma-P-Atom); -22,05 (t, ³J_{PP} = 20,4 Hz, 1P, Beta-P-Atom).

### Beispiel 2

### Synthese von 1-(3'-Amino-2',3'-didesoxy-5'-triphosphat-β-D-threopentofuranosyl)Thymin;

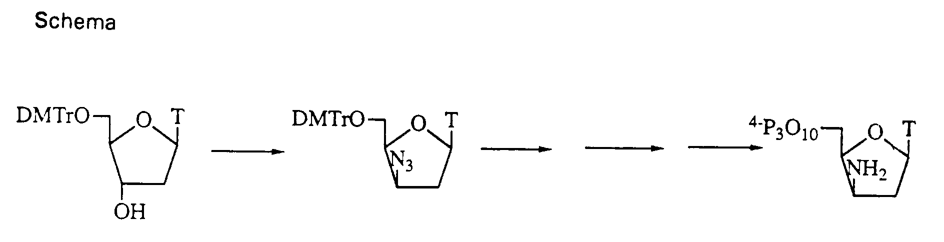

### 1-(3'-Azido-2',3'-didesoxy-5'-O-dimethoxytrityl-β-D-threopentofuranosyl)-Thymin

Zu einer Lösung von 5'-Dimethoxytritylthymidin (Darstellung siehe M. Gait, Oligonucleotide synthesis, IRL Press (1984), S. 27) (5,98 g, 1 1 mmol), Triphenylphosphin (3,076 g, 11,73 mmol) und Lithiumazid (3,1 g, 55 mmoli) in 37 ml trockenem DMF gibt man unter Rühren Kohlenstofftetrabromid (11,91 mmol, 4,10 g) hinzu. Man rührt den Ansatz 56 h bei Raumtemperatur (RT) und fügt dann 15 ml Methanol zu. Das Produkt wird anschließend in 800 ml eiskaltem Aqua dest. präzipitiert. Den Niederschlag nimmt man in Chloroform auf und reinigt durch Flash-Chromatographie (Essigester : n-Hexan = 3 : 1). R_{f}-Wert Chloroform Methanol 9 : 1 = 0,52. IR (cm⁻¹): 2100 Azid, Ausbeute 80 %, 4,95 g.

### 1-(3'-Amino-2',3'-didesoxy-5'-O-dimethoxytrityl-β-D-threopentofuranoxyl)-Thymin

Zu einer gerührten Lösung von 1-(3'-Azido-2',3'-didesoxy-5'-O-dimethoxytrityl-β-D-threopentofuranosyl)-Thymin (0,5 g, 0,8 mmol) gibt man Triphenylphosphin (1,3 g, 4,94 mmol) hinzu und läßt 4 h reagieren. Zum Hydrolysieren des Phosphinimins versetzt man mit 3 ml Aqua dest. und rührt weitere 3 h. Nach dem Abrotieren des Lösungsmittels reinigt man das zurückbleibende Öl durch Flash-Chromatographie (Chloroform : Methanol 99 : 1 + 1 % Triethylamin). R_{f}-Wert im gleichen Laufmittel = 0,27. Das Amin läßt sich durch Behandlung mit Ninhydrin in der Dünnschichtchromatographie (DC) violett anfärben. Ausbeute 87,5 %, 0,42 g. Das Produkt zeigt das entsprechende ¹H-NMR-Spektrum.

### Umsetzung zum 1-(3'-Amino-2',3'-didesoxy-5'-triphosphat-β-D-threopentofuranosyl)-Thymin

Für die weitere Umsetzung zum 5'-Triphosphat wird die Dimethoxytritylgruppe, analog wie bei M. Gait, Oligonucleotide synthesis, IRL Press S. 49 (1984) beschrieben, entfernt und die Ankupplung des Triphosphates an die 5'-Position, wie in Beispiel 1 erwähnt, durchgeführt.

### Beispiel 3

### Synthese von 3'-Amino-2',3'-didesoxy-5'-triphosphat-Adenosin;

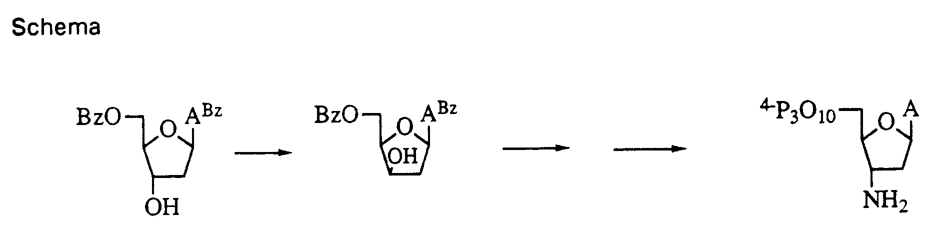

### N⁶-Benzoyl-9-(5-O-benzoyl-2-desoxy-β-D-threo-pentofuranosyl)-Adenin

Eine Suspension von 920 mg (2 mmol) N⁶,5'-O-dibenzoyl-2'-deoxyadenosin (wurde durch die bei Nishino et al., Nucleosides & Nucleotides 1986, 5, 159 beschriebene Methode dargestellt) in 30 ml abs. Dichlormethan (enthält 2 ml Pyridin) wird auf 30°C abgekühlt und langsam 5 ml einer Trifluormethansulfonsäureanhydrid-Lösung in Dichlormethan (10 Vol.-%) zugetropft. Nach dem Entfernen des Kältebades läßt man die Lösung erwärmen und gibt 1 ml Wasser hinzu. Nach 3 h gibt man weitere 5 ml Wasser hinzu und wäscht die organische Phase. Nach dem Entfernen des Lösungsmittels wird der verbleibende Rückstand mit 50 ml Methanol gelöst und 100 mg Natriumbicarbonat zugesetzt. Man rührt weitere 2 h bei RT, neutralisiert mit 10 %iger Essigsäure, destilliert das Lösungsmittel ab und reinigt durch Flash-Chromatographie über Kieselgel auf (Chloroform Methanol 97 : 3). Ausbeute 710 mg (1,48 mmol) 75 %. R_{f}-Wert Chloroform Methanol 97 : 3 = 0,49. MS = 459.

### 3'-Azido-2',3'-didesoxyadenosin

Eine Lösung von 920 mg (2 mmol) N⁶-Benzoyl-9-(5-O-benzoyl-2-deoxy-β-D-threopentofuranosyl)-Adenin in 20 ml abs. Dichlormethan (und 2 ml Pyridin) wird auf 30°C abgekühlt. Dann tropft man 5 ml (3 mmol) einer Trifluormethansulfonsäureanhydridlösung in abs. Dichlormethan hinzu. Man entfernt das Kältebad, rührt weitere 20 min und setzt 980 mg (20 mmol) Lithiumazid in 20 ml DMF zur Lösung. Nach weiteren 2 h Rühren bei RT werden 50 ml Wasser und 150 ml Chloroform zugesetzt, die organische Phase aufgeschüttelt und mit Aqua dest. gewaschen. Man entfernt das Lösungsmittel am ®Rotavapor und behandelt zum Entfernen der Basenschutzgruppe über Nacht mit ammoniakalischer Methanol-Lösung. Nach erneutem Entfernen des Lösungsmittels reinigt man durch Flash-Chromatographie (Chloroform : Methanol 95 : 5) über Kieselgel auf. Ausbeute: 430 mg (1,6 mmol, 79 %) weißes, kristallines Pulver. IR: 2.100 cm⁻¹ Azido-Gruppe.

### 3'-Amino-2',3'-didesoxy-5'-triphosphat-Adenosin

Die Darstellung des 5'-Triphosphates erfolgt analog zum Beispiel 1. Anschließend wird wie in Beispiel 2 das Azid zum Amin reduziert.

### Beispiel 4

### Synthese des 3'-Amino-2',3'-didesoxy-5'-triphosphat-Guanosins;

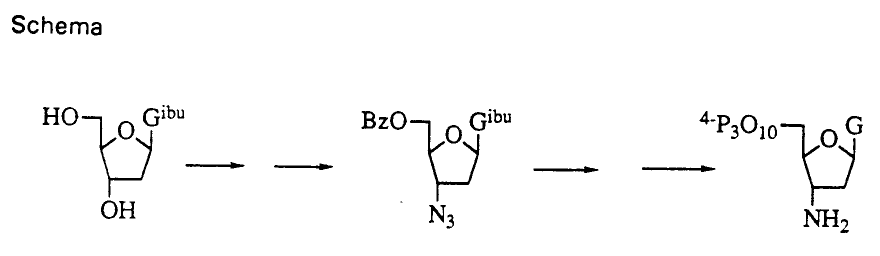

Die Darstellung des 3'-Amino-2',3'-didesoxy-5'-triphosphat-Guanosins erfolgt ausgehend vom N²-Isobutyryl-5'-O-benzoyl-2'-desoxyguanosin, daß sich durch die Methode von Nishino et al. (Nucleosides & Nucleotides 5, 159, 1986) darstellen läßt. Die Umsetzung des Nucleosids zum 3'-Azid erfolgt analog der Darstellung in Beispiel 3. Zu beachten ist hierbei, daß die Behandlung mit ammoniakalischer Methanol-Lösung unterbleibt, weil die Entfernung der Isobutyryl-Schutzgruppe erst nach der Einführung der Triphosphat-Gruppe und vor der Reduzierung des Azids zum Amin stattfindet. Die Umsetzung zum Triphosphat erfolgt auch beim Guanosin nach der in Beispiel 1 erwähnten Methode. Die Reduktion zum Amin ist im Beispiel 2 beschrieben.

### 2. Synthese von 3' -Amino-Oligomeren und nachfolgende 3'-Fluoreszenz-Markierung

### Beispiel 5

Die Synthese eines Oligomers aus 20 Nukleotiden
3'-H₂N-TTTTTTTTTTTTTTTTTTTT-5'
erfolgt ausgehend vom 5'-Dimethoxytrityl geschätzten 3'-Amino-3'-desoxy-Thymidin. Die Darstellung dieser Verbindung verläuft nach der Beschreibung des Beispiels 1. Man geht hierbei von 5'-Dimethoxytrityl geschütztem AZT (Azido-3'-desoxyribosid-Thymidin) aus, welches sich aus AZT und Dimethoxytritylchlorid nach der Methode von M. Gait (Oligonucleotide Synthesis, IRL Press 1984, S. 27) darstellen läßt.

Anschließend wird die Verbindung, wie in Beispiel 1 beschrieben, mit Triphenylphosphin reduziert. Die nächste Stufe ist die Darstellung des Trägermaterials. Hierzu werden 200 mg 5'-0-(4,4'-Dimethoxytrityl)-3'-amino-3'-desoxy-Thymidin in 700 µl abs. Pyridin vorgelegt. Zu dieser Lösung werden 45 mg DMAP (Dimethylaminopyridin) und anschließend 40 mg Bernsteinsäureanhydrid hinzugegeben. Nach Stehenlassen über Nacht hydrolysiert man durch die Zugabe von 10 µl Wasser restliches Bernsteinsäureanhydrid. Man koevaporiert dreimal mit Toluol und nimmt den Rückstand in 12 ml Methylenchlorid auf, wäscht mit 4 ml kalter Zitronensäure (10 %ig) und zweimal mit 4 ml Wasser. Man trocknet die organische Phase über Natriumsulfat, engt das Lösungsmittelvolumen ein und löst die Substanz mit 1 ml Methylenchlorid. Diese Lösung läßt man langsam unter Rühren in 30 ml n-Hexan eintropfen. Den ausgefallenen Niederschlag saugt man ab und trocknet ihn bei 40°C im Ölpumpenvakuum. Die weitere Darstellung des Trägermaterials auf CPG-Basis erfolgt nach Standardprotokollen und zur Darstellung des Oligomers aus 12 Nukleotiden wird mit dem Standardzyklus (ABI 380A User Bulletin, Issue No. 36, Juli 1986) auf einem ABI 380 A DNA-Synthesizer nach dem Phosphoramiditverfahren weitergearbeitet. Nach Entschützung und Abspaltung des 3'-Aminooligonucleotids vom Träger wird standardgemäß auf gereinigt und charakterisiert. Bessere Ergebnisse lassen sich hierbei durch basenlabilere Kupplungsmethoden für die Anbindung an den Träger erzielen.

Günstig ist die Substitution des zu spaltenden Säureamids durch eine Urethan-Funktion, wie dies bei Efimov (Nucleic Acid Res. 11, 8369, 1983) beschrieben wurde.

### Beispiel 6

Die Synthese eines Oligomers aus 23 Nukleotiden mit 3'-Aminoende
3'-H₂N-ACACCCAATTCTGAAAATGGAT-5'
erfolgt nach der Beschreibung in Beispiel 1. Die Aufreinigung und Charakterisierung erfolgt durch Standardmethoden.

### Beispiel 7

Die nachfolgende Derivatisierung der Oligomeren an dem 3'-Amino-Terminus erfolgt mit Fluoresceinisothiocyanat. 50 nmol des 3'-Aminooligonucleotids werden in einem Eppendorf-Gefäß in 25 µl einer 500 mM Natriumhydrogencarbonatlösung aufgelöst. Es werden 20 µl einer 300 mM FITC-Lösung (Sigma) in DMSO zugesetzt. Nach 6 h Reaktionszeit bei Raumtemperatur wird der Reaktionsansatz durch Überleiten in einer 20 mM Ammoniumacetatlösung über eine Sephadex G-25 Säule aufgereinigt. Das 3'-FITC-Oligomer wurde durch einen analytischen HPLC-Lauf sowohl durch Fluoreszenzdetektion als auch durch UV-Detektion analysiert. Die HPLC-Ergebnisse wurden auch durch Analysen auf einer Kapillargelelektrophorese (Firma Dionex) verifiziert.
Formelbild von Fluoresceinisothiocyanat:

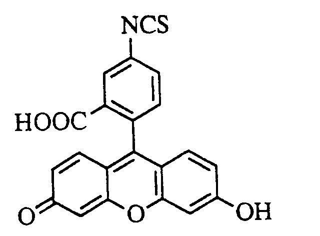

### Beispiel 8

Beide 3'-Aminooligomere aus Beispiel 1 und 2 werden nach der Methode wie in Beispiel 3 beschrieben mit Rhodamin- und Tetramethylrhodamin-isothiocyanat umgesetzt. Die 3'-fluoreszenzmarkierten-Oligomere werden durch einen analytischen HPLC-Lauf sowohl durch Fluoreszenzdetektion als auch durch UV-Detektion analysiert. Die HPLC-Ergebnisse wurden auch durch Analysen auf einer Kapillargelelektrophorese (Firma Dionex) verifiziert.

Formelbilder der Farbstoffe Rhodamin- und Tetramethylrhodaminisothiocyanat

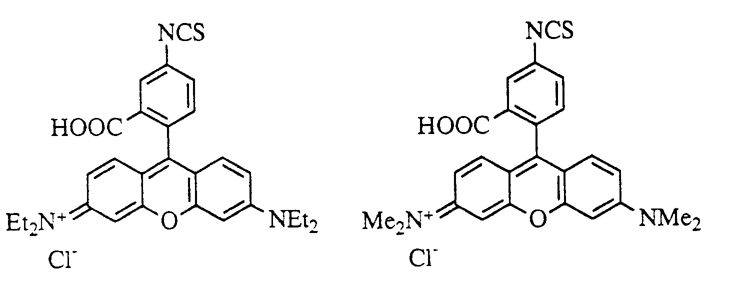

### Beispiel 9

Beide 3'-Aminooligomere aus Beispiel 1 und 2 werden nach der Methode wie in Beispiel 3 beschrieben mit dem abgebildeten Coumarinderivat umgesetzt. Das 3'-fluoreszenzmarkierte Oligomer wird durch einen analytischen HPLC-Lauf sowohl durch Fluoreszenzdetektion als auch durch UV-Detektion analysiert. Die HPLC-Ergebnisse werden auch durch Analysen auf einer Kapillargelelektrophorese (Firma Dionex) verifiziert.

Formelbild des Coumarinfarbstoffmoleküls:

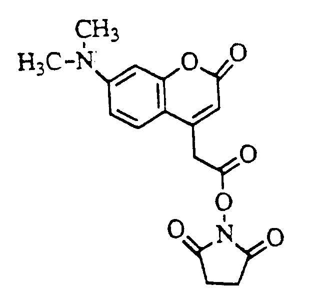

### 3. Einbau modifizierter Nucleotide durch DNA-Polymerasen und Analytik der Sequenzierexperimente

Zur Überprüfung der Akzeptanz der Aminotriphosphate durch die gängigen Sequenzierenzyme wurden entsprechende Terminationslösungen hergestellt und die Polymerasen (Klenow, Boehringer; Taq, Amersham; T7, Pharmacia; Sequenase®, USB) ausgetestet. Hierbei wurden die Einbauraten und die Terminationseigenschaften der Triphosphate sowohl klassisch als auch durch die Markierung mit Fluoreszenzfarbstoffen analysiert.

### Beispiel 10

Das wie Bespiel 1 dargestellte 5'-Triphosphat-3'-amino-3'-desoxyribosid-Thymidin wurde gegen 2',3'-Didesoxy-5'-triphosphat-Thymidin ausgetauscht. Dies erfolgte bei den Terminationslösungen aller oben angegebenen Polymerasen. Es wurden jeweils äquimolare, zehnfach größere und zehnfach geringere dNTP/Terminator-Verhältnisse ausprobiert. Die Sequenzierungen wurden nach Standardprotokollen durchgeführt. Bei diesen Untersuchungen wurde durch den Einbau von alpha-³⁵S-dATP autoradiographisch detektiert. Es zeigt sich, daß a) das 3'-Amino-Nucleotid auf die verwendeten Enzyme terminierend wirkt, b) die Einbauraten bei T7, Taq und Sequenase identisch zu den üblichen Didesoxyterminatoren sind und c) letztendlich mit dem Amino-Nucleotid eine unproblematische Sequenzierung durchgeführt werden kann.

### Beispiel 11

Die Verbindung 5'-Triphosphat-3'-amino-3'-desoxyribosid-Thymidin wurde mit Fluoresceinisothiocyanat zum 5'-Triphosphat-3'-amino-3'-desoxyribosid-3'-N-Fluoresceinisothiocyanat-Thymidin umgesetzt.

5'-Triphosphat-3'-amino-3',2'-desoxyribosid-Thymin-(2,5 mg, 2,8 µmol) wird in einem Eppendorf-Cap in 200 µl Aqua dest. gelöst und mit 200 µl 1 M Na₂C0₃/NaHCO₃-Puffer (pH 9) versetzt. Der Reaktionsansatz wird durch 10 Ummantelung mit Aluminiumfolie vor Tageslicht geschützt und es wird mit einer 100 µl-Pipette 80 µl einer Fluoresceinisothiocyanat-Lösung (10 mg in 1 ml DMF) zugegeben. Nach 1 0 min Reaktionszeit bei Raumtemperatur ist das Edukt im DC quantitativ umgesetzt. Die Aufreinigung erfolgt durch Gelfiltration. Die Sephadex G-10 Säule wird durch Ummantelung mit Aluminiumfolie vor Licht geschützt, anschließend trägt man den kompletten Reaktionsansatz auf das Säulenmaterial auf und eluiert mit einem Fluß von 1 ml/min mit Wasser. Das Chromatogramm zeigt zwei Peaks. Der erste Peak wird durch das Produkt erzeugt und der zweite durch den nicht umgesetzten Farbstoff. Die Fraktionsgröße beträgt 5 ml, ingesamt erhält man 24 Fraktionen. Aufgrund des Chromatogramms erweist sich die Fraktion 4 als positiv. Dies wird durch DC bestätigt. Man engt das Lösungsmittel durch Lyophilisieren ein und lagert die Substanz bei -80°C. Ausbeute 3,32 mg (93 %); MG: 1.274,32; R_{f} (Ammoniak : Isopropanol : Wasser 10 : 70 : 60) = 0,62; Fluoreszenzemissionsspektrum: Die Wellenlänge des eingestrahlten Lichts liegt bei 420 nm. Das Emissionsmaximum der Verbindung liegt bei 514,8 nm. Vermessen wurde eine 2,5 mM Lösung in Aqua dest. Das Emissionsmaximum einer 2,5 mM Lösung des underivatisierten Farbstoffs in Aqua dest. liegt bei einer Wellenlänge von 519,4 nm.

Das dargestellte 5'-Triphosphat-3'-amino-3'-desoxyribosid-3'-N-Fluoresceinisothiocyanat-Thymidin wurde gegen 2',3'-Didesoxy-5'-triphosphat-Thymidin ausgetauscht. Dies erfolgte bei den Terminationslösungen aller oben angegebenen Polymerasen. Es wurden jeweils äquimolare, zehnfach größere und zehnfach geringere dNTP/Terminator-Verhältnisse ausprobiert. Die Sequenzierungen wurden nach Standardprotokollen durchgeführt. Bei diesen Untersuchungen wurde durch den Einbau von alpha-³⁵S-dATP autoradiographisch detektiert. Es zeigt sich, daß a) das 3'-fluoreszenzmarkierte Nucleotid auf die verwendeten Enzyme terminierend wirkt, b) die Einbauraten bei T7, Taq und Sequenase aufgrund des sterisch anspruchsvollen Farbstoffrestes bei zehnfach höheren Konzentrationen zu den üblichen Didesoxyterminatoren identisch sind und c) mit dem 3'-fluoreszenzmarkierten Terminator eine unproblematische Sequenzierung durchgeführt werden kann.

### Beispiel 12

Das dargestellte 5'-Triphosphat-3'-amino-3'-desoxyribosid-3'-N-Fluoresceinisothiocyanat-Thymidin wurde gegen 2',3'-Didesoxy-5'-triphosphat-Thymidin ausgetauscht. Dies erfolgte bei den Terminationslösungen der T7 und Taq Polymerasen. Es wurden zehnfach größere dNTP/Terminator-Verhältnisse zugrundegelegt. Die Sequenzierungen wurden nach Standardprotokollen durchgeführt. Bei diesen Untersuchungen wurde auf den Einbau von alpha-³⁵S-dATP verzichtet und die Sequenzanalyse mit dem 3'-fluoreszenzmarkierten Terminator auf einem kommerziell erhältlichen DNA-Sequenzer erfolgreich durchgeführt.

### Beispiel 13

### Synthese von 5'-Triphosphat-3'-Thio-3'-desoxyribosid-Thymidin

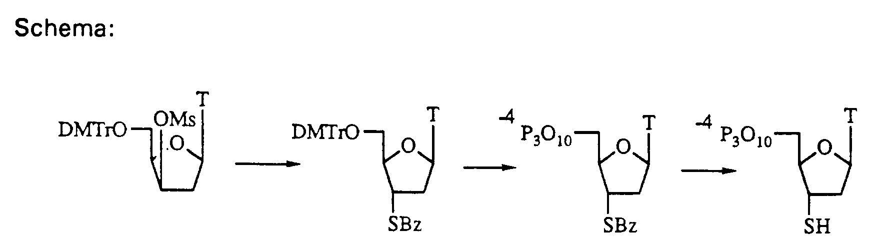

### 5'-O-Dimethoxytrityl-3'-S-benzoyl-thiothymidin

Die Einführung des Schwefels an der 3'-Position erfolgt durch die Substitution einer Abgangsgruppe (Mesylat) mit Natriumthiobenzoat.

Die Synthese von Natriumthiobenzoat erfolgt durch die Zugabe von 10 M NaOH zu einer eisgekühlten Lösung Thiobenzoesäure (10 g) in Wasser 15 ml bis die Lösung gerade alkalisch ist. Anschließend stellt man den pH-Wert auf pH 7 mit wäßriger Thiobenzoesäure ein, kühlt auf -5°C ab und filtriert die Lösung um feste Rückstände zu entfernen. Nach Entfernen des Wassers am Rotationsverdampfer mit Ethanol trocknet man das gelbe Salz im Vacuum über Phosphorpentoxid.
Eine Lösung von 5'-O-Dimethoxytrityl-3'-O-methansulfonyl-2'-deoxyxylothymidin (8,45 mmol) (Synthese des Mesylats: Miller, Fox (1964) J. Org. Chem. 29, 1772) und Natriumthiobenzoat (33 mmol) in DMF (30 ml) wird für 4 h bei 100°C gerührt.
Man schüttelt den Ansatz mit Dichlormethan aus und wäscht die org. Phase mit gesättigter NaHCO₃-Lösung und gesättigter NaCl-Lösung. Nach Trocknen der organischen Phase über Natriumsulfat, koevaporiert man zweimal mit Toluol und reinigt das ölige Rohprodukt durch Flash-Chromatographie (Kieselgel 6OH, Chloroform/Methanol 0-1 0 %).

Ausbeute: 60 %, das Produkt besitzt das erwartete ¹H-NMR-Spektrum.

Die 5'-Dimethoxytritylschutzgruppe wird für die Phosphitilierung an der 5'-Position nach literaturbekannten Methoden (M. Gait, Oligonucleotide Synthesis, IRL-Press, Oxford 1984) abgespalten. Anschließend wird das 5'-Triphosphat nach der Methode von Eckstein und Ludwig (J. Org. Chem., 1989, Vol. 54, No. 3, 631) mit Salicylphosphochloridit (Aldrich) und der Behandlung mit Pyrophosphat hergestellt. Das Thiol wird durch die Reaktion mit 10 M NaOH in Argon gesättigtem Ethanol (R. Cosstick, Nucleic Acids Research 18, 4, 829) freigesetzt.
Das 3'-Thio-5'-Triphosphat-Thymidin wurde nach der Methode in Beispiel 8 mit Jodacetamidofluorescein (Molecular Probes) gekuppelt und man erhält bei diesem Schritt mit 80 %iger Ausbeute das fluoreszenzmarkierte Thionukleotid.

### Beispiel 14

### Synthese von 1-(3'-Thio-2'-,3'-dideoxy-5'-Triphosphat-β-D-threopentofuranosyl)-Thymin

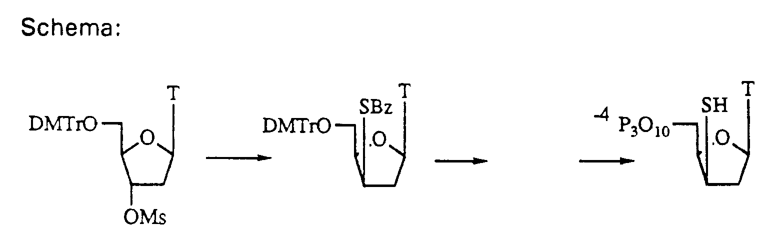

Ausgehend von 5'-DMTr-0-, 3'-O-mesyl-Thymidin wird, wie in Beispiel 14 beschrieben, mit Natriumthiobenzoat substituiert. Anschließend erfolgt die Abspaltung der 5'-Schutzgruppe, die Einführung des 5'-Triphosphates, die Freisetzung des Thiols und die Anbindung von Jodacetamidfluorescein analog den Vorschriften in Beispiel 13.

### Beispiel 15

### Synthese eines Oligomers aus 10 Nukleoliden 3'-β-H₂N-TTT TTT TTT T-5'

Die Synthese eines Oligomers 3'-β-H₂N-TTT TTT TTT T-5' erfolgt ausgehend von 1-(3'-Amino-2',3'-dideoxy-5'-DMTr-β-D-threopentofuranosyl)-Thymin, das nach der Methode wie in Beispiel 6 beschrieben zum Trägermaterial für das Phosphoramiditverfahren umgesetzt wird. Die anschließende Fluoreszenzmarkierung des 3'-β-Amino-Oligonukleotids mit Fluoresceinisothiocyanat erfolgt nach Beispiel 7.

## Patentansprüche (Patentansprüche für folgende(n) Vertragsstaat(en): AT, BE, CH, DE, DK, FR, GB, GR, IT, LI, LU, NL, SE)

1. 3'-Amino- und thiolmodifizierte Nukleoside, Nukleotide und Oligonukleotide der allgemeinen Formel I 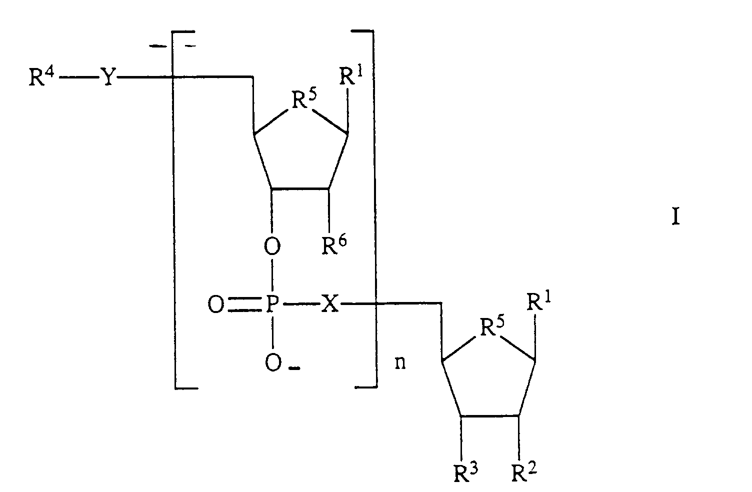 in der
R¹ eine Purin- oder Pyrimidinbase ist,
R³ ein über eine Amino- oder Thiolgruppe gebundener Fluoreszenzfarbstoff in α- oder β-Stellung und
R² Wasserstoff, eine Hydroxyl-, geschützte Hydroxyl- oder Methoxygruppe in α- oder β-Stellung bedeuten, und n eine Zahl von ≧ 0 ist,
R⁴ eine 5'-Schutzgruppe oder Phosphat, Pyrophosphat oder Triphosphat,
R⁵ Sauerstoff, Fluormethylen, Difluormethylen oder Methylen ist,
R⁶ eine Hydroxyl- oder Methoxygruppe oder Wasserstoff in α- oder β-Stellung, wobei R¹, R⁵ und R⁶ jeweils gleiche oder unterschiedliche Bedeutungen haben können,
Y und X Sauerstoff, Schwefel, NH oder Methylen bedeuten,
wobei X und Y jeweils gleich oder verschieden sein können.

2. Verfahren zur Herstellung der Verbindung der Formel I nach Anspruch 1, dadurch gekennzeichnet, daß die in 3'-Stellung befindliche OH-Gruppe eines Nukleosids, Nukleotids oder Oligonukleotids zu einer Amino- oder Thiolgruppe derivatisiert wird und anschließend ein Fluoreszenzfarbstoff angekoppelt wird.

3. Verfahren zur Herstellung der Verbindung der Formel I, dadurch gekennzeichnet, daß in die Verbindung der Formel II 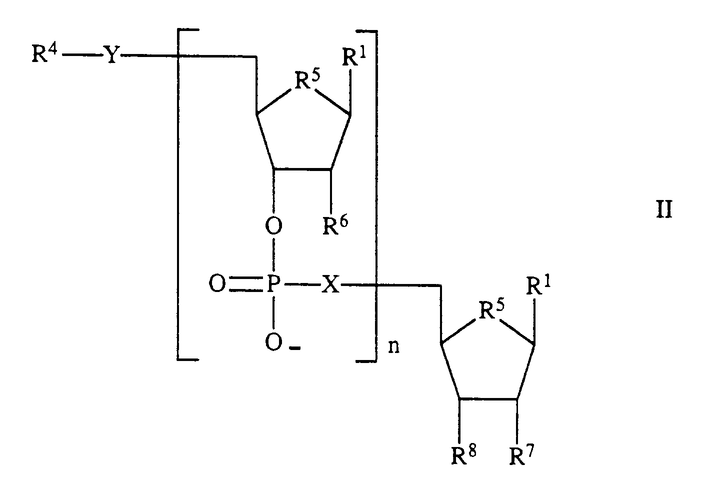 in der R¹-R⁶, X, Y, und n die oben genannten Bedeutungen haben, wobei R¹, R⁵ und R⁶ jeweils gleiche oder unterschiedliche Bedeutungen haben können und R⁷ oder R⁸ Wasserstoff, eine Hydroxyl- oder geschützte Hydroxyl- oder Methoxygruppe bedeuten,
a) in 3'-Stellung durch nukleophilen Angriff ein Azid bzw. ein Thiol in geschützter oder ungeschützter Form eingeführt und gegebenenfalls das Azid zum Amin reduziert und
b) der Fluoreszenzfarbstoff über die Amino- bzw. über die Thiolgruppe gekoppelt wird.

4. Verfahren nach Anspruch 3, dadurch gekennzeichnet, daß als Fluoreszenzfarbstoffe Fluoresceine, Rhodamine, Texasrot, NBD, Coumarine, Fluorescamine, Succinylfluoreszine und Dansyle verwendet werden.

5. Verwendung der Verbindung der Formel I nach Anspruch 1
a) für die Synthese von DNA- und RNA-Nukleosiden, Nukleotiden und Oligonukleotiden
b) für die fluoreszenzmikroskopische und -makroskopische Detektion in vivo und in vitro.

6. Verwendung der Verbindung der Formel I nach Anspruch 5
a) für die Synthese von Gegensträngen in Anwesenheit eines Template-Stranges
b) für die Synthese von Oligonukleotiden.

## Patentansprüche (Patentansprüche für folgende(n) Vertragsstaat(en): ES)

1. Verfahren zur Herstellung der Verbindung der Formel I, 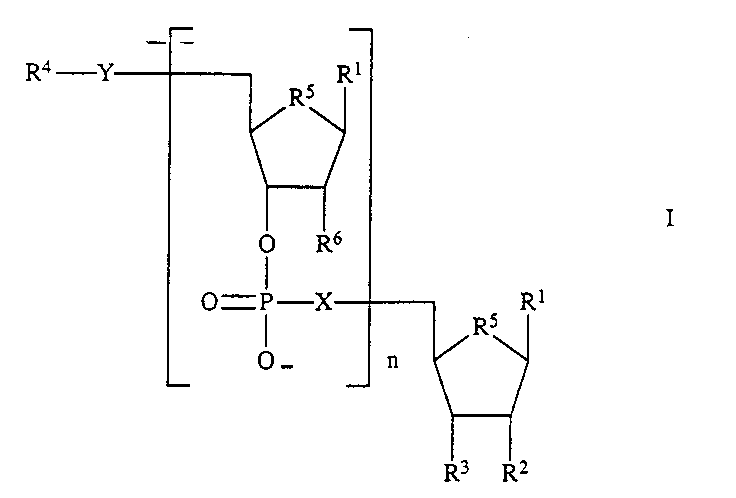 in der
R¹ eine Purin- oder Pyrimidinbase ist,
R³ ein über eine Amino- oder Thiolgruppe gebundener Fluoreszenzfarbstoff in α- oder β-Stellung und
R² Wasserstoff, eine Hydroxyl-, geschützte Hydroxyl- oder Methoxygruppe in α- oder β-Stellung bedeuten, und
n eine Zahl von ≧ 0 ist,
R⁴ eine 5'-Schutzgruppe oder Phosphat, Pyrophosphat oder Triphosphat,
R⁵ Sauerstoff, Fluormethylen, Difluormethylen oder Methylen ist,
R⁶ eine Hydroxyl- oder Methoxygruppe oder Wasserstoff in α- oder β-Stellung, wobei R¹, R⁵ und R⁶ jeweils gleiche oder unterschiedliche Bedeutungen haben können,
Y und X Sauerstoff, Schwefel, NH oder Methylen bedeuten,
wobei X und Y jeweils gleich oder verschieden sein können, dadurch gekennzeichnet, daß die in 3'-Stellung befindliche OH-Gruppe eines Nukleosids, Nukleotids oder Oligonukleolids zu einer Amino- oder Thiolgruppe derivatisiert wird und anschließend ein Fluoreszenzfarbstoff angekoppelt wird.

2. Verfahren zur Herstellung der Verbindung der Formel I nach Anspruch 1, dadurch gekennzeichnet, daß die in 3'-Stellung befindliche OH-Gruppe eines Nukleosids, Nukleotids oder Oligonukieotids zu einer Amino- oder Thiolgruppe derivatisiert wird, indem zuerst an der 3'-Hydroxylgruppe eine Leaving-Gruppe eingeführt wird, sodann der nucleophile Angriff des Azids zur Bildung des Azids bzw. der nucleophile Angriff des Thiolats oder S-geschützten Thiolats zur Bildung des Thiols oder S-geschützten Thiols erfolgt und anschließend die freie 3'-Amino- oder Thiolgruppe des Zuckerteils am 3' Ende der Nucleinsäure mit einem reaktiven Fluoreszenzfarbstoff reagiert.

3. Verfahren zur Herstellung der Verbindung der Formel I, dadurch gekennzeichnet, daß in die Verbindung der Formel II 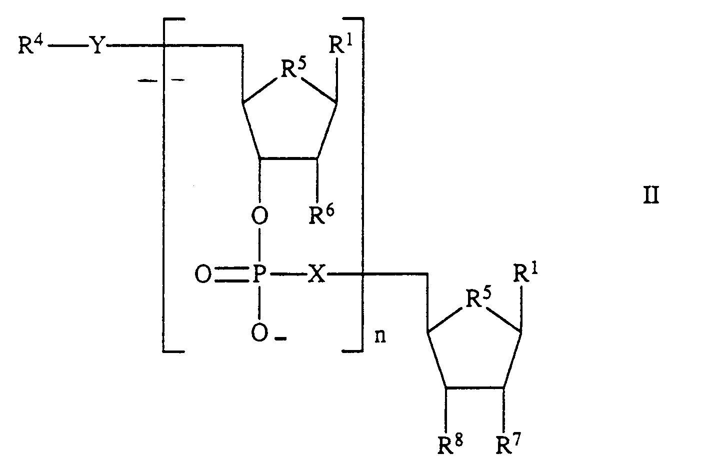 in der R¹-R⁶, X, Y, und n die oben genannten Bedeutungen haben, wobei R¹, R⁵ und R⁶ jeweils gleiche oder unterschiedliche Bedeutungen haben können und R⁷ oder R⁸ Wasserstoff, eine Hydroxyl- oder geschützte Hydroxyl- oder Methoxygruppe bedeuten,
a) in 3'-Stellung durch nukleophilen Angriff ein Azid bzw. ein Thiol in geschützter oder ungeschützter Form eingeführt und das Azid zum Amin reduziert und
b) der Fluoreszenzfarbstoff über die Amino- bzw. über die Thiolgruppe gekoppelt wird.

4. Verfahren nach den Ansprüchen 1-3, dadurch gekennzeichnet, daß als Fluoreszenzfarbstoffe Fluoresceine, Rhodamine, Texasrot, NBD, Coumarine, Fluorescamine, Succinylfluoreszine und Dansyle verwendet werden.

5. Verwendung der gemäß Anspruch 1 hergestellten Verbindung
a) für die Synthese von DNA- und RNA-Nukleosiden, Nukleotiden und Oligonukleotiden
b) für die fluoreszenzmikroskopische und -makroskopische Detektion in vivo und in vitro.

6. Verwendung der gemäß Anspruch 1 hergestellten Verbindung
a) für die Synthese von Gegensträngen in Anwesenheit eines Template-Stranges
b) für die Synthese von Oligonukleotiden.

## Claims (Claims for the following Contracting State(s): AT, BE, CH, DE, DK, FR, GB, GR, IT, LI, LU, NL, SE)

1. A 3'-amino- and thio-modified nucleoside, nucleotide or oligonucleotide of the formula I 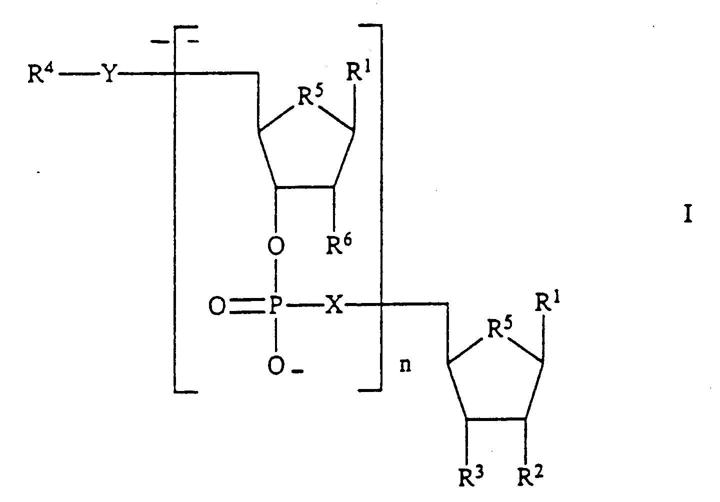 in which
R¹ is a purine or pyrimidine base,
R³ is a fluorescent dye in α or β position bonded via an amino or thiol group, and
R² is hydrogen, a hydroxyl, protected hydroxyl or methoxy group in α or β position, and n is a number ≧ 0,
R⁴ is a 5' protective group or phosphate, pyrophosphate or triphosphate,
R⁵ is oxygen, fluoromethylene, difluoromethylene or methylene,
R⁶ is a hydroxyl or methoxy group or hydrogen in α or β position, where R¹, R⁵ and R⁶ can in each case have identical or different meanings,
Y and X are oxygen, sulfur, NH or methylene,
where X and Y can in each case be identical or different.

2. A process for the preparation of the compound of the formula I as claimed in claim 1, which comprises derivatizing the OH group located in the 3' position of a nucleoside, nucleotide or oligonucleotide to an amino or thiol group, and subsequently coupling on a fluorescent dye.

3. A process for the preparation of the compound of the formula I, which comprises in the compound of the formula II 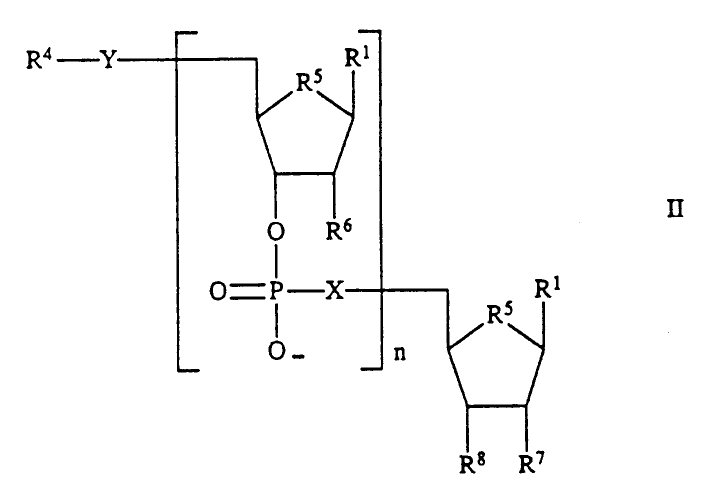 in which R¹-R⁶, X, Y and n have the abovementioned meanings, where R¹, R⁵ and R⁶ can in each case have identical or different meanings, and R⁷ or R⁸ are hydrogen, a hydroxyl or protected hydroxyl or methoxy group,
a) introducing an aside or a thiol in protected or unprotected form in the 3' position by nucleophilic attack and, where appropriate, reducing the azide to the amine and
b) coupling the fluorescent dye via the amino group or via the thiol group.

4. The process as claimed in claim 3, wherein fluoresceins, rhodamines, Texas red, NBD, coumarins, fluorescamines, succinylfluoresceins and dansyls are used as fluorescent dyes.

5. The use of the compound of the formula I as claimed in claim 1
a) for the synthesis of DNA and RNA nucleosides, nucleotides and oligonucleotides
b) for microscopic and macroscopic fluorescence detection in vivo and in vitro.

6. The use of the compound of the formula I as claimed in claim 5
a) for the synthesis of complementary strands in the presence of a template strand
b) for the synthesis of oligonucleotides.

## Claims (Claims for the following Contracting State(s): ES)

1. A process for the preparation of the compound of the formula I 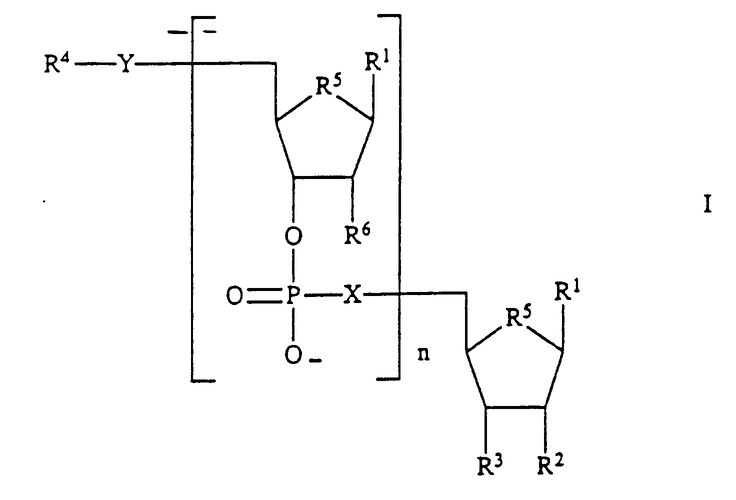 in which
R¹ is a purine or pyrimidine base,
R³ is a fluorescent dye in α or β position bonded via an amino or thiol group, and
R² is hydrogen, a hydroxyl, protected hydroxyl or methoxy group in α or β position, and
n is a number ≧ 0,
R⁴ is a 5' protective group or phosphate, pyrophosphate or triphosphate,
R⁵ is oxygen, fluoromethylene, difluoromethylene or methylene,
R⁶ is a hydroxyl or methoxy group or hydrogen in α or β position, where R¹, R⁵ and R⁶ can in each case have identical or different meanings,
Y and X are oxygen, sulfur, NH or methylene,
where X and Y can in each case be identical or different, which comprises derivatizing the OH group located in the 3' position of a nucleoside, nucleotide or oligonucleotide to an amino or thiol group, and subsequently coupling on a fluorescent dye.

2. A process for the preparation of the compound of the formula I as claimed in claim 1, which comprises derivatizing the OH group located in the 3' position of a nucleoside, nucleotide or oligonucleotide to an amino or thiol group by first introducing a leaving group on the 3'-hydroxyl group, then the nucleophilic attack of the azide is carried out to form the azide or the nucleophilic attack of the thiolate or S-protected thiloate is carried out to form the thiol or S-protected thiol and subsequently the free 3'-amino or thiol group of the sugar portion at the 3' end of the nucleic acid reacts with a reactive fluorescent dye.

3. A process for the preparation of the compound of the formula I, which comprises, in the compound of the formula II 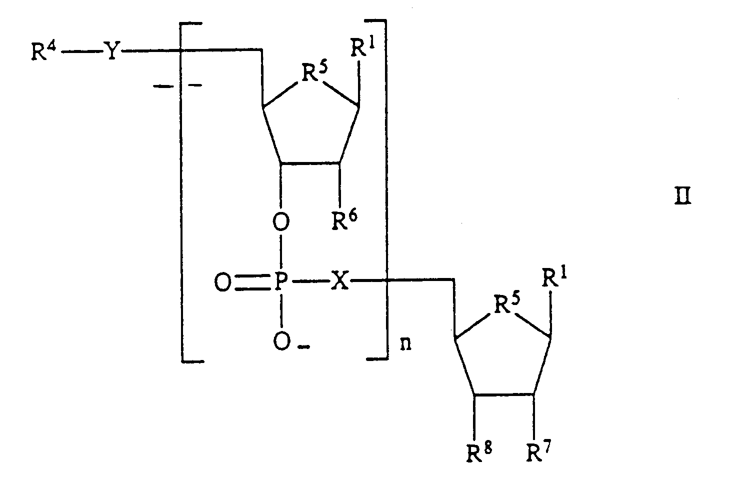 in which R¹-R⁶, X, Y and n have the abovementioned meanings, where R¹, R⁵ and R⁶ can in each case have identical or different meanings, and R⁷ or R⁸ are hydrogen, a hydroxyl or protected hydroxyl or methoxy group,
a) introducing an azide or a thiol in protected or unprotected form in the 3' position by nucleophilic attack and reducing the azide to the amine and
b) coupling the fluorescent dye via the amino group or via the thiol group.

4. The process as claimed in claims 1-3, wherein fluoresceins, rhodamines, Texas red, NBD, coumarins, fluorescamines, succinylfluoresceins and dansyls are used as fluorescent dyes.

5. The use of the compound prepared as claimed in claim 1
a) for the synthesis of DNA and RNA nucleosides, nucleotides and oligonucleotides
b) for microscopic and macroscopic fluorescence detection in vivo and in vitro.

6. The use of the compound prepared as claimed in claim 1
a) for the synthesis of complementary strands in the presence of a template strand
b) for the synthesis of oligonucleotides.

## Revendications (Revendications pour l'(les) Etat(s) contractant(s) suivant(s): AT, BE, CH, DE, DK, FR, GB, GR, IT, LI, LU, NL, SE)

1. Nucléosides, nucléotides et oligonucléotides à modification 3'-amino et thiol, de formule générale I 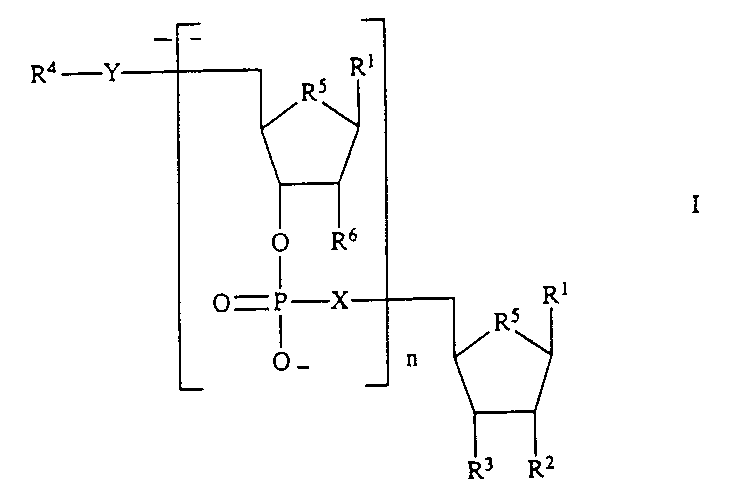 dans laquelle
R¹ est une base purique ou pyrimidique,
R³ représente un colorant fluorescent, en position α ou β, lié par un groupe amino ou thiol, et
R² représente un atome d'hydrogène ou un groupe hydroxy, hydroxy protégé ou méthoxy, en position α ou β, et
n est un nombre ≧ 0,
R⁴ représente un groupe protecteur en 5' ou le groupe phosphate, pyrophosphate ou triphosphate,
R⁵ est un atome d'oxygène ou le groupe fluorométhylène, difluorométhylène ou méthylène,
R⁶ représente un atome d'hydrogène ou un groupe hydroxy ou méthoxy en position α ou β, R¹, R⁵ et R⁶ pouvant avoir des significations identiques ou différentes,
Y et X représentent un atome d'oxygène ou de soufre, ou le groupe NH ou méthylène,
X et Y pouvant être identiques ou différents.

2. Procédé pour la préparation du composé de formule I selon la revendication 1, caractérisé en ce que le groupe OH présent en position 3' d'un nucléoside, nucléotide ou oligonucléotide est transformé en un groupe amino ou thiol, couplé ensuite avec un colorant fluorescent.

3. Procédé pour la préparation du composé de formule I, caractérisé en ce que, dans le composé de formule II 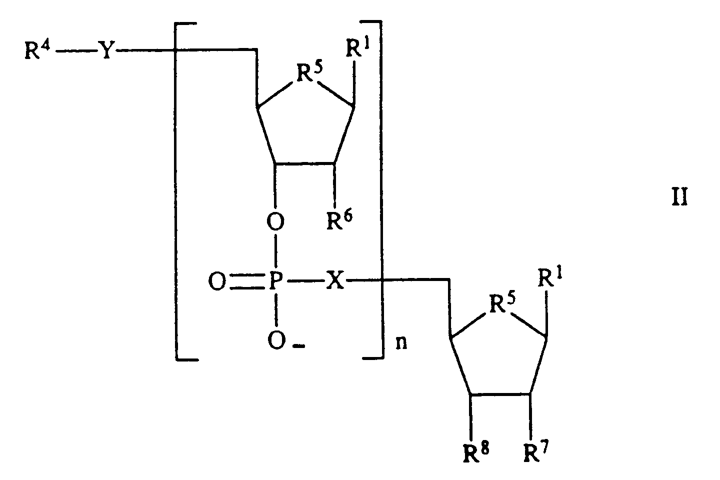 dans laquelle R¹-R⁶, X, Y et n ont les significations données plus haut, R¹, R⁵ et R⁶ pouvant avoir des significations identiques ou différentes, et R⁷ ou R⁸ représentent un atome d'hydrogène ou un groupe hydroxy, hydroxy protégé ou méthoxy,
a) un azide ou un thiol, sous forme protégée ou non protégée, est introduit en position 3' par attaque nucléophile, et éventuellement l'azide est réduit en amine, et
b) le colorant fluorescent est couplé par l'intermédiaire du groupe amino ou du groupe thiol.

4. Procédé selon la revendication 3, caractérisé en ce l'on utilise comme colorants fluorescents des fluorescéines, des rhodamines, le rouge Texas, le NBD, des coumarines, fluorescamines, succinylfluorescéines et des dansyles.

5. Utilisation du composé de formule I selon la revendication 1
a) pour la synthèse de nucléosides, nucléotides et oligonucléotides d'ADN et d'ARN,
b) pour la détection microscopique et macroscopique par fluorescence in vivo et in vitro.

6. Utilisation du composé de formule I selon la revendication 5
a) pour la synthèse de brins complémentaires en présence d'un brin matrice,
b) pour la synthèse d/oligonucléotides.

## Revendications (Revendications pour l'(les) Etat(s) contractant(s) suivant(s): ES)

1. Procédé pour la préparation du composé de formule I, 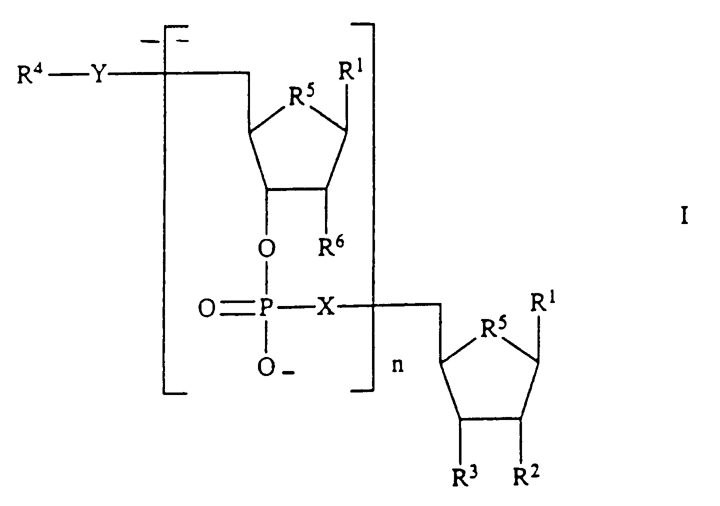 dans laquelle
R¹ est une base purique ou pyrimidique,
R³ représente un colorant fluorescent, en position α ou β, lié par un groupe amino ou thiol, et
R² représente un atome d'hydrogène ou un groupe hydroxy, hydroxy protégé ou méthoxy, en position α ou β, et
n est un nombre ≧ 0,
R⁴ représente un groupe protecteur en 5' ou le groupe phosphate, pyrophosphate ou triphosphate,
R⁵ est un atome d'oxygène ou le groupe fluorométhylène, difluorométhylène ou méthylène,
R⁶ représente un atome d'hydrogène ou un groupe hydroxy ou méthoxy en position α ou β, R¹, R⁵ et R⁶ pouvant avoir des significations identiques ou différentes,
Y et X représentent un atome d'oxygène ou de soufre, ou le groupe NH ou méthylène,
X et Y pouvant être identiques ou différents,
caractérisé en ce que le groupe OH présent en position 3' d'un nucléoside, nucléotide ou oligonucléotide est transformé en un groupe amino ou thiol, couplé ensuite avec un colorant fluorescent.

2. Procédé pour la préparation du composé de formule I selon la revendication 1, caractérisé en ce que l'on transforme en un groupe amino ou thiol le groupe OH présent en position 3' d'un nucléoside, nucléotide ou oligonucléotide en introduisant d'abord un groupe partant sur le groupe hydroxy en 3', puis en effectuant l'attaque nucléophile de l'azide pour la formation de l'amine, ou l'attaque nucléophile du thiolate ou du thiolate à S protégé pour la formation du thiol ou du thiol à S protégé, et on fait ensuite réagir avec un colorant fluorescent réactif le groupe 3'-amino ou -thiol libre du fragment glucidique à l'extrémité 3' de l'acide nucléique.

3. Procédé pour la préparation du composé de formule I, caractérisé en ce que, dans le composé de formule II 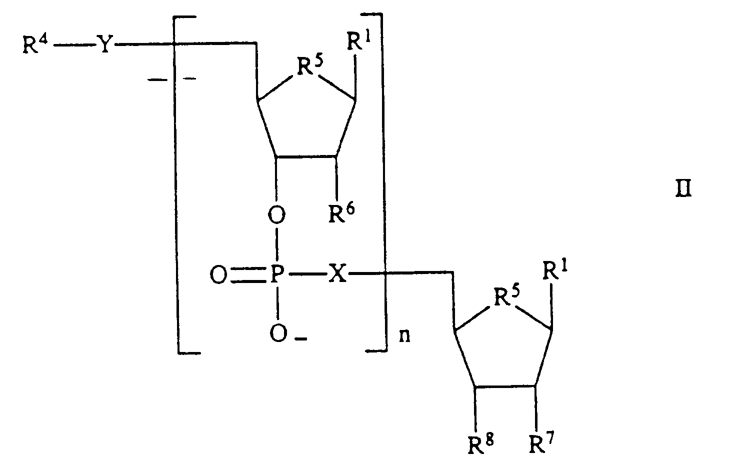 dans laquelle R¹-R⁶, X, Y et n ont les significations données plus haut, R¹, R⁵ et R⁶ pouvant avoir des significations identiques ou différentes, et R⁷ ou R⁸ représentent un atome d'hydrogène ou un groupe hydroxy, hydroxy protégé ou méthoxy,
a) un azide ou un thiol, sous forme protégée ou non protégée, est introduit en position 3' par attaque nucléophile, et l'azide est réduit en amine, et
b) le colorant fluorescent est couplé par l'intermédiaire du groupe amino ou du groupe thiol.

4. Procédé selon les revendications 1 à 3, caractérisé en ce l'on utilise comme colorants fluorescents des fluorescéines, des rhodamines, le rouge Texas, le NBD, des coumarines, fluorescamines, succinylfluorescéines et des dansyles.

5. Utilisation du composé préparé selon la revendication 1
a) pour la synthèse de nucléosides, nucléotides et oligonucléotides d'ADN et d'ARN,
b) pour la détection microscopique et macroscopique par fluorescence in vivo et in vitro.

6. Utilisation du composé préparé selon la revendication 1
a) pour la synthèse de brins complémentaires en présence d'un brin matrice,
b) pour la synthèse d'oligonucléotides.
